# EUROPÄISCHE PATENTSCHRIFT

(11) **EP 2 368 533 B1**
(45) Veröffentlichungstag und Bekanntmachung des Hinweises auf die Patenterteilung: **04.12.2013**
(21) Anmeldenummer: 11002068.2
(22) Anmeldetag: 12.03.2011
(51) Int. Cl.: A61F 13/15

(54) **Hygieneartikel mit Funktionseinzelumverpackung**
Sanitary item with individual functional packaging
Article hygiénique doté d'un emballage double unique à fonction

(30) Priorität: 22.03.2010 DE 102010012272
(43) Veröffentlichungstag der Anmeldung: 28.09.2011
(73) Patentinhaber: Fleischmann, Stefan, 44807 Bochum (DE); Blome, Richard, 44807 Bochum (DE); Rintjema, Frans, 44807 Bochum (DE); Ihmann, Axel, 44807 Bochum (DE)
(72) Erfinder: Fleischmann, Stefan, 44807 Bochum (DE); Blome, Richard, 44807 Bochum (DE); Rintjema, Frans, 44807 Bochum (DE); Ihmann, Axel, 44807 Bochum (DE)

(56) Entgegenhaltungen:
- EP-A1- 2 243 450
- US-A- 4 581 027
- US-A- 4 857 066
- US-A- 5 413 568
- US-A1- 2007 049 891

## Beschreibung

Die Erfindung betrifft einen Hygieneartikel, insbesondere eine Inkontinenzeinlage, Slipeinlage oder Damenbinde, mit einer Einzelumverpackung, wobei die Einzelumverpackung und der Hygieneartikel eine Einheit bilden und die Einzelumverpackung den Hygieneartikel ganz umschließt, vor Verunreinigung schützt und nach Gebrauch dazu geeignet ist, den kontaminierten Hygieneartikel geruchs- und feuchtigkeitshemmend zu umschließen.

Stand der Technik sind Hygieneartikel, mit Einzelumverpackung wobei die Verpackung vor Gebrauch des Hygieneartikels zum Schutz desselben dient und nach Gebrauch als Entsorgungsverpackung den kontaminierten Hygieneartikel geruchs- und feuchtigkeitshemmend umschließt. So wird zum Beispiel in der europäischen Patentschrift EP 0 656 772 B1 eine Erfindung beschrieben, die die vorgenannten Merkmale aufweist.

Beim Stand der Technik besteht das Problem, dass aufgrund der Art und Weise der Falttechnik eine Handhabung des Hygieneartikels ohne Berührung der Hände mit den kontaminierten Flächen nicht sicher gewährleistet werden kann. Insbesondere bei fehlender, ebener Ablagefläche in der freien Natur oder räumlich, beengten Verhältnissen ist die gebrauchsanleitungsgemäße Anwendung der beschriebenen Falttechnik nur schwer durchführbar.

Darüber hinaus führt die anwendungsgerechte Falttechnik in der Regel zu einer Kompression der kontaminierten Absorptionsflächen, was zum Austritt von kontaminierten Bestandteilen aus dem Hygieneartikel führen kann.

Als eine besondere Schwachstelle bei herkömmlicher Falttechnik erweisen sich hier die Knickachsen, in deren Endpunkten sich die Haftstreifen durch die Faltung derart stauchen, dass ein sicheres Verschließen nicht gewährleistet werden kann.

Ein weiterer Stand der Technik ist die Hygieneeinlage der Patentschrift US 4 857 066 A, welche eine Verpackung vor und nach der Benutzung beinhaltet Vor der Benutzung ist die Schutzverpackung an den Enden geschlossen und muss entfernt werden. Nach Benutzung werden beide Flügel zum Überlappen des benutzte Hygienekörpers genutzt.

Ein weiterer Stand der Technik ist das United States Patent 4,581,027,welches eine integrierte Entsorgungsumverpackung nach Benutzung beinhaltet. Ein verschließender Klebestreifen, welcher die Hygieneeinlage in der Unterwäsche fixiert, muss zur Benutzung freigelegt werden. Durch das Umstülpen der unten liegenden Taschen wird die benutzte Fläche abgedeckt. Durch die Überstreckung der Taschen wird der Hygieneartikel verschlossen.

Ein weiterer Stand der Technik ist das United States Patent US 5 413 568 A, welches eine Hygieneeinlage beinhaltet, die durch das Aufklappen von Faltungen geöffnet wird. In der Transportumverpackung ist die Hygienefläche sicher verschlossen.

Ein weiterer Stand der Technik ist das United States Patent US 2007/049891 A1, bei dem es sich um ein mehrteiliges System mit Transport- und Entsorgungs-verpackung handelt. Klebestreifen zur Fixierung in der Unterwäsche, werden hier durch Schutzstreifen abgedeckt.

Ein weiterer Stand der Technik ist das epa Patent EP 2 243 450 A1, welches durch das Aufklappen von Faltungen gleich dem Patent US 5 413 568 A geöffnet wird. Im Transportzustand ist die Hygienefläche verschlossen. Aufgabe unserer Erfindung ist es, den eingangs beschriebenen Hygieneartikel derart zu gestalten, dass die hygienische Sicherheit bei einfacher, praktischer Handhabung gewährleistet wird.

Diese Aufgabe wird mit einem Hygieneartikel der eingangs bezeichneten Art gelöst, bei dem die Einzelumverpackung auf der Körperseite des Hygieneartikels einen Eingriff mit überlappenden Rändern aufweist, die vor Gebrauch des Hygieneartikels auf dessen körperabgewandte Seite umschlagbar sind und nach Gebrauch wieder in die ursprüngliche Position rückführbar sind.

Die nach Anspruch 1 formulierte Erfindung löst die Aufgabe dergestalt, dass der Funktionszustand des Hygieneartikels durch einen einfachen Handgriff verändert werden kann. Die Einzelumverpackung übernimmt hierbei zwei Funktionen. Vor Gebrauch schützt sie die Absorptionsfläche vor Verunreinigung von außen. Nach Gebrauch verschließt sie die Absorptionsfläche mit ihren kontaminierten Bestandteilen geruchs- und feuchtigkeitshemmend. Während des Gebrauchs ist der körperseitige Teil der Einzelumverpackung auf die Rückseite (körperabgewandte Seite) umgeschlagen und gibt den Absorptionsbereich frei.

Erfindungsgemäß ist die Einzelumverpackung durch einen einfachen Handgriff von dem Verpackungszustand in den Benutzungszustand und anschließend wieder in den Verschlusszustand überführbar, indem das Innere nach außen beziehungsweise das Äußere nach Innen gewendet wird.

Durch den Verzicht auf Knickachsen beim Verschließen nach Gebrauch des Hygieneartikels bei zusätzlichem Einsatz einer überlappenden Schutzhülle, die mit Haftstreifen versehen ist, wird - verglichen mit dem Stand der Technik - eine verbesserten Dichtigkeit erzeugt.

Die Erfindung kann in allen Bereichen der Hygieneeinlagen (Damenbinden, Slipeinlagen, Inkontinenzeinlagen, Tierhygiene) angewandt werden.

Ein Vorteil der Erfindung liegt in der einfachen und schnellen Handhabung.

Der erfindungsgemäße Hygieneartikel weist insbesondere einen Absorptionsbereich mit einem Absorptionsmaterial auf.

Ein weiterer Vorteil liegt in der hohen Verschlussdichte der Einzelumverpackung vor Gebrauch. So ist der Absorptionsbereich vor Verunreinigung geschützt und sorgt für eine hohe hygienische Sicherheit. Auf eine sonst zusätzlich notwendige Einzelverpackung kann deshalb verzichtet werden.

Aus der Handhabung des Hygieneartikels ergibt sich ein weiterer Vorteil bezüglich der hygienischen Sicherheit, da der Hygieneartikel bei der Handhabung lediglich an den Enden berührt werden muss und keine weiteren Hilfsmittel wie zum Beispiel Ablageflächen nötig sind. So ist ein Kontakt zwischen den kontaminierten Bestandteilen und den Händen des Nutzers bzw. der Nutzerin weitgehend vermeidbar. Die Einzelumverpackung kann dazu an den Enden Markierungspunkte aufweisen.

Gegenüber dem Stand der Technik, der das nachträgliche Verschließen durch Falten erreicht, bietet die Erfindung den Vorteil, dass eine ungewollte selbstständige Öffnung nach Gebrauch durch "Zurückfaltung" ausgeschlossen wird.

Darüber hinaus ist der Hygieneartikel äußerst einfach von seinem Aufbau her konstruiert und kostengünstig herzustellen.

Gegenüber Hygieneartikeln des Standes der Technik, die vor dem Gebrauch bereits gefaltet sind, oder vorgeformte Faltachsen besitzen, bietet die Erfindung einen Komfortvorteil, da solche Verformungen bzw. Stauchungen in den Knickachsen die Oberfläche der Absorptionsfläche verändern und so Reibungsflächen erzeugen. Der unveränderte, dem Körper angepasste Zustand ist bei der Erfindung vor Gebrauch gegeben, eine Formveränderung durch Falten ist nicht nötig.

Bei der Verwendung von Haftstreifen ergibt sich aufgrund der Konstruktion als weiterer Vorteil, dass keine zusätzlichen Schutzstreifen benötigt werden, da die Haftstreifen in keinem der Funktionszustände offen liegen und so an ungewollten Stellen haften bleiben könnten. So gibt es keine zusätzlichen Teile, die entsorgt werden müssten und für eine umständliche Handhabung sorgen, da beim Austausch einer benutzten Einlage durch eine neue, die zu entfernenden Schutzstreifen zunächst beiseite gelegt werden müssen um die Hände für die Positionierung der neuen Einlage frei zu haben und anschließend wieder aufgenommen werden müssen, um sie der Entsorgung zuzuführen.

Ein weiterer Vorteil ergibt sich aus der Tatsache, dass die Einzelumverpackung sich bei Gebrauch des Hygieneartikels in doppelter und überlappender Lage zwischen Absorptionsfläche und Kleidungsstück befindet und so für einen erhöhten Schutz der Kleidungsstücke vor Verunreinigung sorgt.

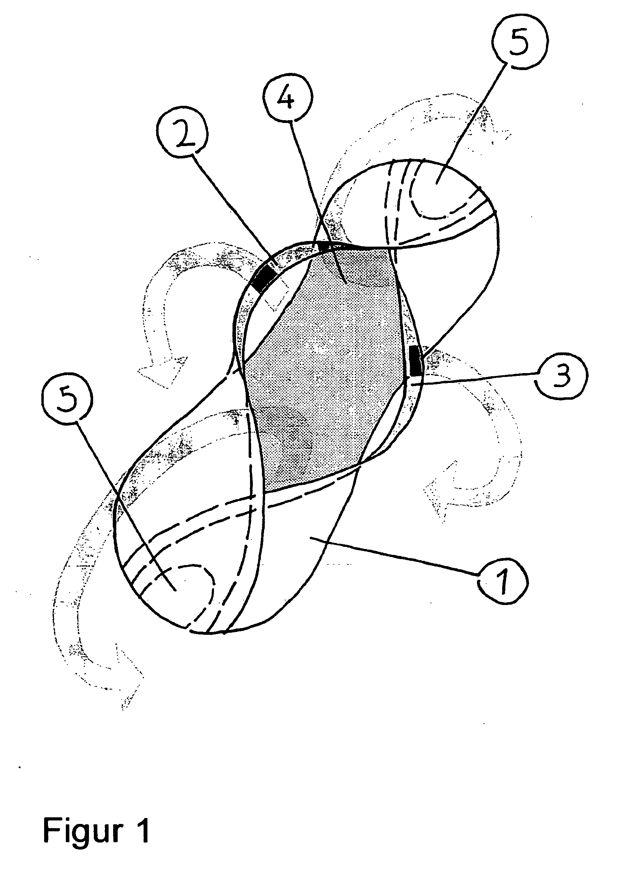

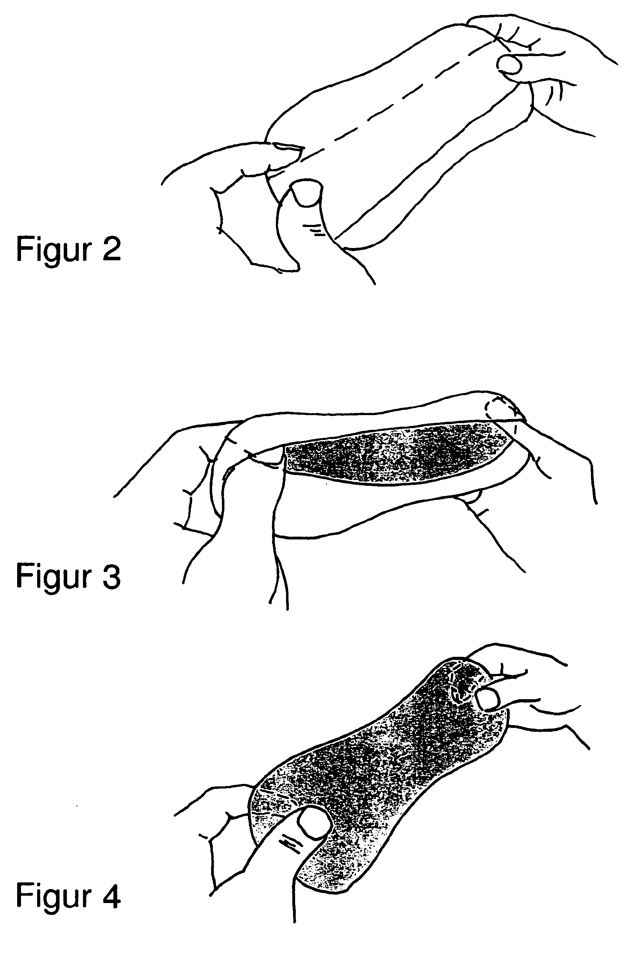

Die Figur 1 zeigt eine mögliche Ausführung der Erfindung. Vor Gebrauch des Hygieneartikels wird der Absorptionsbereich (4) durch die sich überlappenden Ränder der Einzelumverpackung bzw. Schutzhülle (1) vor Verunreinigungen von außen geschützt. Der Haftstreifen (2) innerhalb der Überlappung der Schutzhülle (1) verschließt die Verpackung zusätzlich. Es versteht sich, dass die Schutzhülle (1) auch die körperabgewandte Seite des Hygieneartikels abdeckt und dort fixiert ist.

Um den Hygieneartikel in seinen Gebrauchszustand zu versetzen, wird die Schutzhülle (1) im Bereich des Schlitzes oder Eingriffs mit den überlappenden Rändern entsprechend den dargestellten Pfeilen umgestülpt bzw. umgeschlagen. Die Markierungspunkte (5) markieren die Stellen, an denen die Daumen auf der einen Seite, und die Zeige- und Mittelfinger auf der anderen Seite, aufgesetzt werden. Die Zeige- und Mittelfinger gleiten zwischen die sich überlappenden Ränder der Schutzhülle (1) und lösen so den Haftstreifen (2). Durch leichten Druck der Daumen unterstützt durch das Hochziehen der Finger wird die Absorptionsfläche (4) freigelegt. Die Ränder der Schutzhülle (1), die zuvor den Absorptionsbereich (4) bedeckt hatten, stülpen sich dadurch auf die andere, dem Körper abgewandten Seite.

Die Ränder der Einzelumverpackung (1) befinden sich nun in doppelter und überlappender Lage auf der körperabgewandten Seite. Die Absorptionsfläche (4) ist somit freigelegt und vorbereitet für die Aufnahme von Flüssigkeiten. Der Haftstreifen (2), der zuvor die Schutzfolien (1) verschlossen hat, fixiert nun den sich überlappenden Bereich der Schutzfolien auf der anderen, körperabgewandten Seite. Der Haftstreifen (3), der zuvor innen, auf der Absorptionsfläche (4) haftete, befindet sich nun auf der Außenseite und dient der Fixierung des Hygieneartikels am Kleidungsstück.

Nach Gebrauch des Hygieneartikels wird dieser zunächst von dem Kleidungsstück gelöst. Hierbei markieren die Markierungspunkte (5) wieder die Positionen, an der die Daumen auf der einen Seite, und die Zeige- und Mittelfinger auf der anderen Seite, aufgesetzt werden. Nun werden die Schutzfolien (1) nach dem gleichen Schema wie zuvor wieder zurückgestülpt, sodaß der Ursprungszustand wieder hergestellt ist und der nun kontaminierte Absorptionsbereich (4) wieder von den Schutzfolien (1) umschlossen wird.

Der Haftstreifen (2) verschließt wieder den überlappenden Bereich der Schutzfolien (1). Der kontaminierte Absorptionsbereich (4) ist somit komplett von der Einzelumverpackung umschlossen und kann entsorgt werden bzw. bis zur endgültigen Entsorgung mitgeführt werden.

Der erfindungsgemäße Hygieneartikel kann weitere Haft- und Fixierelemente aufweisen, die die Festlegung an einem Kleidungsstück ermöglichen oder verbessern, etwa Klettelemente, oder aber die Fixierung im gefaltetem oder eingerolltem Zustand ermöglichen. Ferner kann er einen Indikator aufweisen, der anzeigt, ob der Hygieneartikel neuwertig oder gebraucht ist.

## Patentansprüche

1. Hygieneartikel, insbesondere Inkontinenzeinlage, Slipeinlage oder Damenbinde, mit einer Einzelumverpackung (1), wobei die Einzelumverpackung (1) und der Hygieneartikel eine Einheit bilden und die Einzelumverpackung (1) den Hygieneartikel ganz umschließt, vor Verunreinigung schützt und nach Gebrauch dazu geeignet ist, den kontaminierten Hygieneartikel geruchs- und feuchtigkeitshemmend zu umschließen, **dadurch gekennzeichnet, dass** die Einzelumverpackung (1) auf der Körperseite des Hygieneartikels einen Eingriff mit überlappenden Rändern aufweist, die vor Gebrauch des Hygieneartikels auf dessen körperabgewandte Seite umschlagbar sind und nach Gebrauch wieder in die ursprüngliche Position rückführbar sind.

2. Hygieneartikel nach Anspruch 1, **dadurch gekennzeichnet, dass** er ein Absorptionsmaterial aufweist.

3. Hygieneartikel nach Anspruch 1 oder 2, **dadurch gekennzeichnet, dass** die Umverpackung (1) ein oder mehrere Haftelemente (2, 3) an den überlappenden Rändern aufweist.

4. Hygieneartikel nach Anspruch 3, **dadurch gekennzeichnet, dass** das oder die Haftelemente (2) geeignet sind, die Einzelumverpackung (1) derart zu verschließen, dass der Hygieneartikel vor Gebrauch vor Verunreinigungen geschützt ist und nach Gebrauch erneut verschließt, so dass der Hygieneartikel geruchs- und feuchtigkeitshemmend umschlossen ist.

5. Hygieneartikel nach Anspruch 3 oder 4, **dadurch gekennzeichnet, dass** das oder die Haftelemente (3) geeignet sind, den Hygieneartikel an einem Kleidungsstück zu fixieren.

6. Hygieneartikel nach einem der vorstehenden Ansprüche, **dadurch gekennzeichnet, dass** er über ein oder mehrere Fixierelemente (3) verfügt, die geeignet sind, den Hygieneartikel an einem Kleidungsstück zu fixieren.

7. Hygieneartikel nach einem der Ansprüche 3 bis 6, **dadurch gekennzeichnet, dass** er über ein oder mehrere Haftelemente verfügt, die geeignet sind, den Hygieneartikel nach einer Formveränderung durch Falten, Knicken oder Rollen zu fixieren.

8. Hygieneartikel nach einem der vorstehenden Ansprüche, **dadurch gekennzeichnet, dass** er auf der Umverpackung Markierungen (5) als Handhabungshilfe aufweist.

9. Hygieneartikel nach einem der vorstehenden Ansprüche, **dadurch gekennzeichnet, dass** er einen Indikator aufweist, der geeignet ist, den Gebrauchszustand anzuzeigen.

10. Hygieneartikel nach einem der vorstehenden Ansprüche, **dadurch gekennzeichnet, dass** die Einzelumverpackung (1) aus einem elastischen Material besteht, das sich in entspanntem Zustand derart zusammenzieht, dass der Kontakt zwischen Einzelumverpackung (1) und Haut des Nutzers bzw. der Nutzerin minimiert wird.

## Claims

1. Sanitary item particularly incontinence pads, panty liners or sanitary towels, with an individual functional packing (1), where at the separate packing (1) and the sanitary item form a unitary item and the separate packing (1) completely envelopes the sanitary item, protects against contamination and is suitable to envelope the contaminated sanitary time in an odour-inhibiting and damp-resistant way after usage, **characterized in that** the separate packing (1) contains an access with overlapping edges on the body side of the sanitary item, which can be turned up onto the reverse side of the body before using the sanitary item and can be turned back into the original position after using the sanitary item.

2. Sanitary item according to claim 1 **characterized in that** it contains an absorbent material.

3. Sanitary item according to claim 1 or 2 **characterized in that** it contains one or more sticky elements (2, 3) of the individual functional packing (1) on the overlapping edges.

4. Sanitary item according to claim 3 **characterized in that** the one or more sticky elements (2) is suited to enclose the individual functional packing (1) in such a way that the sanitary item is protected against contamination before usage and enclose the sanitary item again after usage so that the sanitary item is enveloped in an odour-inhibiting and damp-resistant way.

5. Sanitary item according to claim 3 or 4 **characterized in that** the sticky element(s) (3) is suited to fasten the sanitary item to a piece of clothing.

6. Sanitary item according to one of the above-mentioned claims **characterized in that** it contains one or more fixing elements (3) which are suited to fasten the sanitary item to a piece of clothing.

7. Sanitary item according to one of the claims 3 to 6 **characterized in that** the one or more sticky elements (2, 3) is suitable to keep the sanitary item in shape after changing shape by folding, creasing or winding.

8. Sanitary item according to one of the above-mentioned claims **characterized in that** it contains markings (5) as a handling guidance on the individual functional packing (1).

9. Sanitary item according to one of the above-mentioned claims **characterized in that** it contains an indicator which shows the currant usage condition.

10. Sanitary item according to one of the above-mentioned claims **characterized in that** the individual functional packing (1) consists of flexible material which in a slackened state contracts in such a way that the contact between the individual functional packing (1) and the skin of the user is minimized.

## Revendications

1. Article d'hygiène, notamment protection contre l'incontinence, protège-slip ou serviette hygiénique pourvue d'une enveloppe individuelle (1), cette enveloppe individuelle (1), et l'article d'hygiène forment un ensemble, l'enveloppe individuelle (1) enrobe complètement l'article d'hygiène, le protège de la contamination et après utilisation, elle est appropriée à envelopper l'article d'hygiène contaminé, de manière à renfermer les odeurs et l'humidité, la caractéristique de l'enveloppe individuelle (1) étant, que du côté corps se trouve une ouverture avec des bords rabattables qui avant utilisation de l'article hygiénique se rabattent vers le côté opposé au corps et après utilisation se replient dans la position initiale.

2. Article d'hygiène selon la revendication 1, **se caractérisant par** l'existence d'une matière absorbante.

3. Article d'hygiène selon la revendication 1 ou 2, **se caractérisant par** l'enveloppe (1) comportant un ou plusieurs éléments adhésifs (2, 3) sur les bords rabattables.

4. Article d'hygiène selon la revendication 3, **se caractérisant par** l'existence d'un ou plusieurs éléments adhésifs (2) appropriés à fermer l'enveloppe individuelle (1) de manière qu'avant utilisation l'article d'hygiène soit protégé contre la contamination et se referme après utilisation, enveloppant ainsi l'article d'hygiène et renfermant les odeurs et l'humidité.

5. Article d'hygiène selon les revendications 3 ou 4, **se caractérisant par** le ou les éléments adhésifs (3) adaptés à fixer l'article d'hygiène à un vêtement.

6. Article d'hygiène selon une des revendications précédentes, **se caractérisant par** l'existence d'un ou plusieurs éléments de fixation (3) adaptés à fixer l'article d'hygiène à un vêtement.

7. Article d'hygiène selon les revendications 3 à 6, **se caractérisant par** l'existence d'un ou plusieurs éléments adhésifs adaptés à fixer l'article d'hygiène en modifiant sa forme en le pliant, le roulant ou le recourbant.

8. Article d'hygiène selon une des revendications précédentes, **se caractérisant par** l'existence sur l'emballage de conditionnement de marquages (5) comme conseil d'utilisation.

9. Article d'hygiène selon une des revendications précédentes, **se caractérisant par** l'existence d'un indicateur destiné à indiquer l'état d'utilisation.

10. Article d'hygiène selon une des revendications précédentes, **se caractérisant par** l'enveloppe individuelle (1) constituée d'une matière souple qui - à l'état détendu - se contracte de sorte à ce que le contact entre l'enveloppe individuelle (1) et la peau de l'utilisateur, de l'utilisatrice soit minimisé.
